# EUROPEAN PATENT SPECIFICATION

(11) **EP 0 639 978 B1**
(45) Date of publication and mention of the grant of the patent: **19.03.1997**
(21) Application number: 92910125.1
(22) Date of filing: 12.05.1992
(51) Int. Cl.: A61K 33/24

(54) **PHARMACEUTICAL COMBINATION HAVING ANTINEOPLASTIC ACTIVITY**
PHARMAZEUTISCHE MISCHUNG MIT ANTINEOPLASTISCHER WIRKSAMKEIT
COMBINAISON PHARMACEUTIQUE PRESENTANT UNE ACTIVITE ANTINEOPLASIQUE

(30) Priority: 16.05.1991 IT MI001344
(43) Date of publication of application: 01.03.1995
(73) Proprietor: ISTITUTO NAZIONALE PER LA RICERCA SUL CANCRO, I-16132 Genova (IT)
(72) Inventor: ESPOSITO, Mauro, I-16132 Genova (IT)
(74) Representative: Minoja, Fabrizio
(86) International application number: EP9201038
(87) International publication number: WO9220353

(56) References cited:
- EP-A- 0 305 008
- US-A- 4 536 386

## Description

The present invention relates to a pharmaceutical combination having antineoplastic activity based on platinum complexes and procainamide.

Cisplatin (DDP) is one of the most effective and most used drugs in the antineoplastic therapy, and is the precursor of a new class of drugs having a potent antitumor activity, such as carboplatin, tetraplatin, iproplatin, TNO-6-, DACH and the like (Chemistry in Britain, Nov. 86, pp 1001-1004). The use of such drugs is however limited by the severe toxic and side effects (nephrotoxicity, neurotoxicity, nausea, emesis and hemopoietic toxicity). The therapeutic strategies adopted to limit DDP and other platinum complex toxicity to date brought no real therapeutic advantages. It has been evidenced how the protection from toxic effects is often associated to a reduction of oncolytic activity, which is not balanced by the possibility to administer higher doses of the drug.

In the attempt to limit DDP toxicity, without however limiting antitumor efficacy, the combination between DDP and procaine (P.HCl), a well-known local anesthetic, was proposed; nevertheless, the obtained results were not particularly encouraging, since the therapeutic index of said combination showed an insufficient improvement (Esposito M. et al., J Natl Cancer Inst 8 : 677, 1990).

It has now surprisingly been found that the combination between DDP or other antitumor platinum complexes and procainamide hydrochloride (Pd.HCl), a well-known antiarithmic agent, not only shows a longer and significantly higher activity in comparison with the procaine combination, even in terms of survival, but also allows to obtain a remarkable protection from the typical toxicity of platinum complexes.

By itself, procainamide has no antitumor activity, but, when combined with DDP, it protects particularly from acute nephrotoxicity, hemopoietic toxicity, mitochondrial damage, oxidative phosphorylation and other unwanted metabolic effects, which are induced by the antiblastic agent, further improving the therapeutic activity thereof.

According to the invention, the combination exerts its effects both by simultaneous use of the two components, and by separate use of the same.

Therefore, the present invention relates both to a combination of DDP or other platinum complexes with Pd.HCl, the two components to be contemporaneously administered by parenteral or intracavitary administration, and to a kit containing the two drugs, in the form of two distinct pharmaceutical compositions to be separately administered, optionally even by different ways of administration, procainamide being administrable also by oral route.

The specific administration route is not critical for the pharmaceutical combination efficacy.

The weight ratio between the platinum complex and Pd.HCl is comprised between 1:15 and 1:2, preferably between 1:10 and 1:4, most preferably about 1:6.

The pharmaceutical combination of the present invention can be prepared in formulations suitable for the parenteral or intracavitary administration, such as for example injectable aqueous or saline solutions, the latter having a suitable concentration. In the case of kits for the separate administration of the two drugs, they can contain the platinum complex in the form of the above parenteral or intracavitary formulations and the procainamide in the form of parenteral, intracavitary or oral formulations. Examples of oral formulations are solid forms, such as tablets, sugar-coated pills, gelatin capsules and the like, or liquid forms, such as syrups, drinkable ampoules, solutions, etc.

The pharmaceutical formulations can be prepared according to methods well-known in the art, such as for example those described in "Remington's Pharmaceutical Sciences Handbook", Mack Publish. Co., N.Y, USA.

The invention is further described in the following experimental section, wherein specific reference to DDP is made.

However, analogous results can be obtained also with the other above cited platinum complexes.

The study of the Pd.HCl effect on DDP toxicity and antitumor efficacy comprises some experiments carried out with the two drugs, which were dissolved both into aqueous solutions (evaluation of the combination effect on renal toxicity) and into NaCl solutions, which allow to evaluate the combination efficacy under conditions of chloride ion concentrations equivalent to physiological saline (0.9%).

### Hystological evaluation of renal damage

Microscopy analysis of the samples obtained from kidney of animals treated with 8 mg/kg of DDP alone at days 1, 5 and 9 (high toxicity condition) revealed renal parenchyma alterations, which were localized at the cortical zone level. Stromal connective, wherein rare and scattered fibroblasts were detected, appeared remarkably thickened with resulting intertubular space increase. Renal corpuscle morphology appeared in a good preservation state in the components thereof and a slight increase of the glomerular chamber was recognized in few corpuscles only.

The exam of the proximal convoluted tubule epithelium permitted to recognize an epithelium reduction with resulting tubular lumen increase. Besides epithelium flattening, a remarkable upsetting of the brush border, which appeared shortened with scarse, or in same portions, even absent villi.

The analysis of the samples treated with Pd.HCl only (50 mg/kg on days 1, 5 and 9) showed a picture similar to the one observed with DDP only, with resulting increase and confluence of the areas having tubular reduction.

To summarize, a high tubular damage, induced by the two individually administered drugs, was observed in the samples obtained from a three-days treatment.

The analysis of DDP-Pd.HCl combination (8 mg of DDP/kg - 50 mg of Pd-HCl/kg) showed pictures significantly different. The tubules, showing the above described structural alterations of the surface epithelium, resulted remarkably decreased and were found in those areas with a relative tubular reduction, as a consequence of slight damages induced by an associated treatment with DDP-Pd.HCl using the same doses, which proved to be extremely toxic when the two drugs were individually administered.

The following tables and the enclosed figures refer to experiments carried out on BDFl mice inoculated with P388 leukemic cells and wherein the effect of DDP-Pd.HCl combination is compared, in terms of toxicity and therapeutic efficacy, with the one of the different components. From Pd-HCl toxicity results, which are reported in Table 1, the 50 mg/kg of Pd.HCl dose was selected for the investigations suited for the evaluation of DDP-Pd.HCl combination toxicity and therapeutic efficacy.

Examples referring to the Pd.HCl induced protection against DDP acute toxicity and nephrotoxicity are reported in Tables 2 and 3 and in Figure 1a, b, c. Tables 4 and 5 report examples referred to Pd.HCl efficacy on DDP therapeutic activity. In Figure 2, relative survival curves evidence that in survival terms there is no difference between simultaneous or separate DDP-Pd.HCl treatment, whereas for both of them a significant survival increase is registered with respect to the treatment with DDP only. Survival distributions of animals treated with DDP-Pd.HCl or an equivalent dose of DDP-P.HCl are reported in Figure 3. The higher efficacy of Pd.HCl compared to P.HCl in improving DDP therapeutic index is evident.

**TABLE 1**

| Effect of several Pd.HCl doses on leukemic mice mortality and body weight.^{a)} | | | |
|---|---|---|---|
| Pd.HCl (mg/kg/IP) | N. | Survivors on day 14 (%) | Weight variation on day 7 (%) |
| 50 | 8 | 8 (100) | - 0,18 |
| 100 | 8 | 8 (100) | - 2,44 |
| 150 | 8 | 8 (100) | - 2,53 |
| 200 | 8 | 8 (100) | - 4,28 |

| | | | |
|---|---|---|---|
| ^{a)} BDFl female mice inoculated with 10⁵ P388 leukemic cells/mouse i.p. on day 0 and i.p. treated 24 hours (day 1) after inoculation with different doses of Pd.HCl in 0.1-0.2 ml of distilled water. | | | |

**TABLE 2**

| Effect of simultaneous administration of Pd.HCl on mortality and body weight leukemic mice treated with several DDP aqueous solutions.^{a)} | | | | |
|---|---|---|---|---|
| DDP (mg/kg/IP) | Pd.HCl (mg/kg/IP) | N. | Survivors on day 14 (%) | Weight variation on day 7 (%) (Mean±S.D.) |
| 8 | - | 14 | 12 (86) | -17,2±1,9 |
| 12 | - | 14 | 4 (29) | -23,6±1,4 |
| 16 | - | 14 | 1 (7) | -^{b} |
| 25 | - | 14 | 0 (0) | -^{b} |
| 8 | 50 | 14 | 14 (100) | - 3,9±2,1 |
| 12 | 50 | 14 | 14 (100) | - 4,0±1,8 |
| 16 | 50 | 14 | 14 (100) | - 6,5±2,9 |
| 25 | 50 | 14 | 14 (100) | -16,1±2,4 |
| 32 | 50 | 14 | 4 (29) | -21,6±3,0 |

| | | | | |
|---|---|---|---|---|
| ^{a)} see Table 1 explanation | | | | |
| ^{b)} 90% mortality on days 5 or 6. | | | | |

**TABLE 3**

| Protective effect of Pd.HCl on DDP-induced acute nephrotoxicity in P388 leukemic mice (N=8/group). Pd.HCl and DDP were i.p. administered at the same time. Urea, expressed as ureic nitrogen (BUN), was determined from day 1 to day 8 after the treatment. | | | | |
|---|---|---|---|---|
| Day | BUN (mg/100 ml, Mean±S.D.) in the groups:^{a)} | | | |
| | DDP (mg/kg) | | DDP (mg/kg)+Pd.HCl (50 mg/kg) | |
| | 8 | 16 | 8 | 16 |
| 1 | 20,0±1,2 | 19,4±5,9 | 13,8±1,5 | 12,8±1,5 |
| 2 | 25,5±3,1 | 20,7±0,7 | 17,8±2,7 | 27,0±5,0 |
| 3 | 31,3±7,8 | 34,2±1,5 | 18,6±0,9^{b} | 22,3±1,2^{c} |
| 4 | 42,9±0,7 | 93,5±59,0 | 21,7±2,1^{c} | 24,8±4,1^{c} |
| 5 | 48,6±1,9 | 44,8±22,3 | 21,9±0,6^{c} | 34,7±7,7 |
| 6 | 59,1±33,9 | 26,2±9,7 | 15,6±5,0^{c} | 27,1±0,8 |
| 7 | 109,8±42,8 | ND^{d} | 16,6±2,7^{c} | 20,1±3,3 |
| 8 | 28,6±0,5 | ND^{d} | 16,5±2,0^{b} | 24,2±2,2 |

| | | | | |
|---|---|---|---|---|
| ^{a)} untreated control mice BUN = 18.8±1.3 mg/100 ml. | | | | |
| ^{b)} P<0.05 (Student's t test) compared to the mice treated with the same amount of DDP only. | | | | |
| ^{c)} P<0.01 (Student's t test) compared to the mice treated with the same amount of DDP only. | | | | |
| ^{d)} ND= not detectable, 100% mortality on day 7. | | | | |

**TABLE 4**

| Influence of Pd.HCl on DDP therapeutic activity in P388 leukemia treatment in BDFl female mice. | | | | | |
|---|---|---|---|---|---|
| Treatment^{a)} | | | MST^{b} (day) | ILS^{c} (%) | Survivors on day 60 (alive/treated) |
| DDP (mg/kg/IP) | Pd.HCl (mg/kg/IP) | Vehicle | | | |
| Day 1 | | | | | |
| 16 | - | H2O | 5 | -58 | 0/10 |
| 16 | - | 0,9% NaCl | 22 | 83 | 2/10 |
| 16 | 50 | H2O | >60 | >400 | 10/18 |
| 16 | 50 | 0,63% NaCl | 43 | 258 | 10/20 |

| Days 1 and 5 | | | | | |
|---|---|---|---|---|---|
| 8 | - | H2O | 7 | -42 | 0/8 |
| 8 | 50 | H2O | >60 | >400 | 5/8 |

| | | | | | |
|---|---|---|---|---|---|
| ^{a)} Treatment protocol: i) single treatment (16 mg/kg of DDP) starting 24 hours after leukemic cell inoculation (10⁵ P388 cell/mouse, i.p.). Pd.HCl was i.p. administered at the same time with DDP in water or a saline having final Cl⁻ ion concentration corresponding to a 0.9% NaCl solution (Esposito, vide supra); ii) twice repeated treatment (8 mg/kg of DDP administered 24 and 120 hours after leukemic cell inoculation. | | | | | |
| ^{b)} MST = median survival time. In control mice, MST was only 12 days for the untreated ones, 13 days for the Pd.HCl only treated ones. | | | | | |
| ^{c)} Survival increase in comparison with untreated controls. | | | | | |

**TABLE 5**

| I.V. administered Pd.HCl influence on i.p. administered DDP therapeutic activity in P388 leukemia treatment in BDFl female mice. | | | | |
|---|---|---|---|---|
| DDP^{a} (mg/kg/IP) | Pd.HCl^{b} (mg/kg/IP) | MST^{a} (day) | ILS^{a} (%) | Survivors on day 60 (alive/treated) |
| - | 50 in H2O | 13 | 8,3 | 0/8 |
| 16 in H2O | 50 in 0,63% NaCl | >60 | >400 | 5/8 |
| 16 in 0,63% NaCl | 50 in H2O | >60 | >400 | 5/8 |

| | | | | |
|---|---|---|---|---|
| ^{a)} See Table 4 explanation. | | | | |
| ^{b)} Pd.HCl was i.v. administered immediately after DDP. | | | | |

The activity of the association of the present invention is further illustrated by the enclosed figures.

### Particularly:

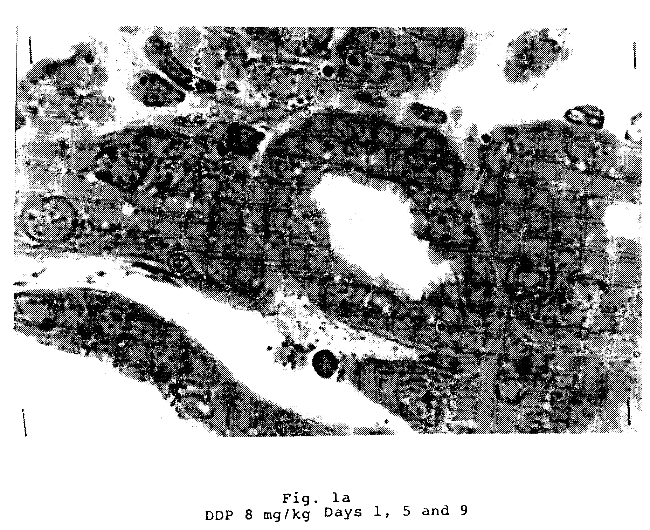

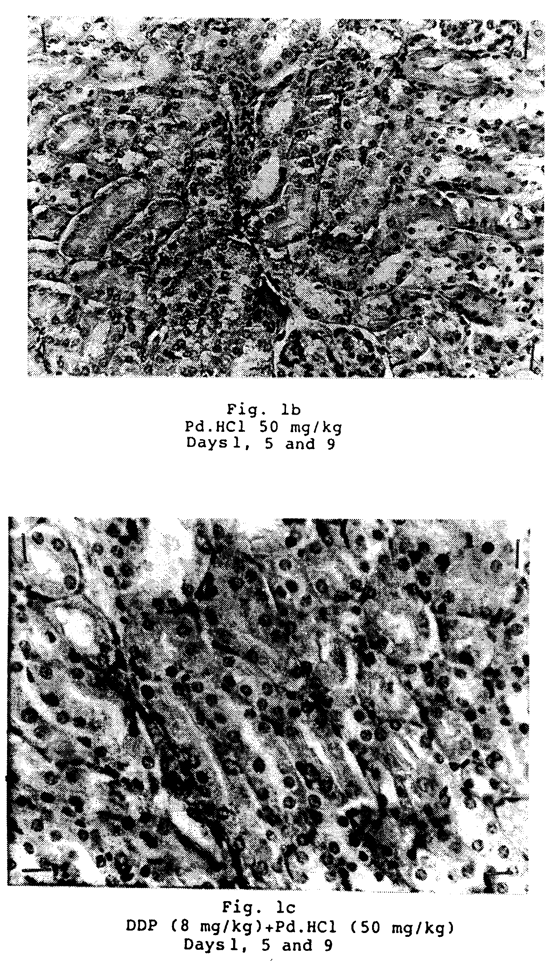

Figure 1 shows the extension of tubular damage in kidneys of P388 leukemic BDFl mice, which were killed the 10^{th} day after a treatment with whether (a) DDP (8 mg/kg) in days 1, 5 and 9, or (b) Pd.HCl (50 mg/kg), or (c) the combination of DDP (8 mg/kg) - Pd.HCl (50 mg/kg) in distilled water (Magnification 400x). Immediately after withdrawal, renal parenchyma was fixed with 2.5% glutaraldehyde in 0.1 M Na cacodylate buffer (pH = 7.4) for 120 min.

After washing in the same buffer, the samples were dehydrated with ethanol and embedded in Epon 812. LKB Ultratom III microtome 2 µm sections were stained with aqueous 1% toluidine blue.

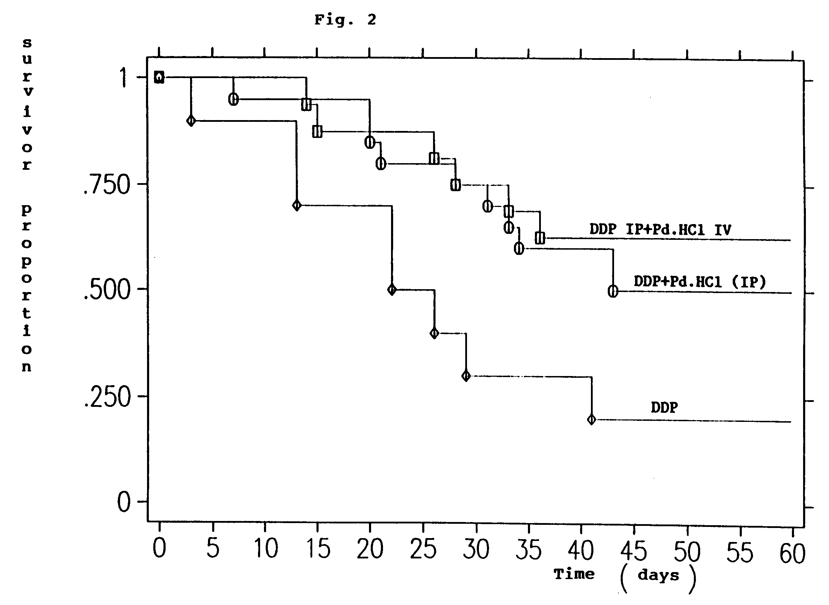

Figure 2 shows the survival curves in P388 leukemic BDFl mice treated with DDP only (16 mg/kg in 0.9% NaCl (◇)), with DDP + 50 mg/kg of Pd.HCl i.p. given simultaneously in 0.63% NaCl (○) and with DDP i.p. given + 50 mg/kg of Pd.HCl i.v. given in 0.63% NaCl (□) (see Tables 4 and 5).

Both simultaneous i.p. treatment and separate administration evidence a significant survival increase with respect to the treatment with DDP only (P=0.037 and P=0.0176, respectively, according the generalized Wilcoxon's test).

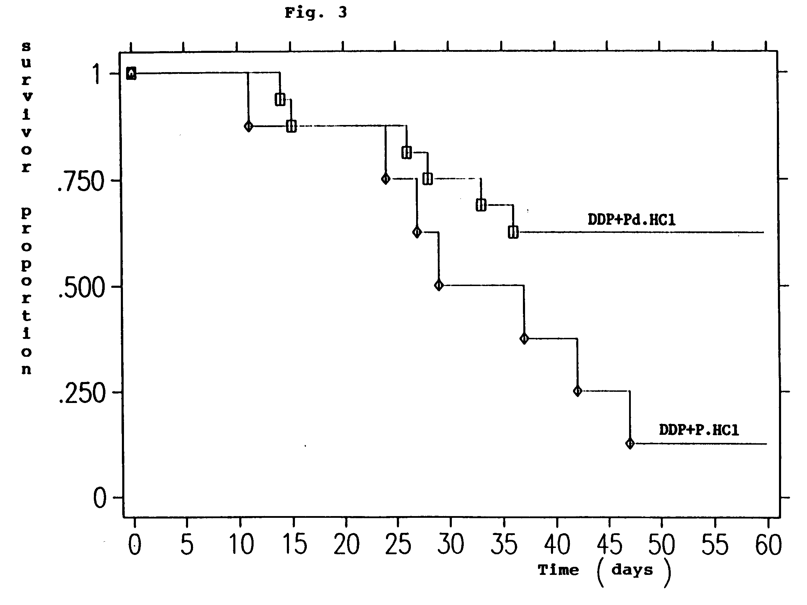

Figure 3 reports survival curves in P388 leukemic BDFl mice treated with DDP i.p. given (16 mg/kg) + Pd.HCl i.v. given (50 mg/kg) in 0.63% HCl (◇) or with DDP i.p. given (16 mg/kg) + P.HCl i.v. given (40 mg/kg), in 0.69% NaCl (◇).

The statistical analysis between the survival distribution of the animals treated with DDP+Pd.HCl and with DDP+P.HCl shows that Pd.HCl induced a significant increase of survival with respect to P.HCl (P=0.0397).

## Claims

1. Pharmaceutical combination having antineoplastic activity which contains cisplatin or other antitumor platinum complexes and procainamide.

2. Pharmaceutical combination according to claim 1, in the form of aqueous or saline solution to be administered through parenteral or intracavitary route.

3. Pharmaceutical combination according to claim 1, in the form of a kit containing cisplatin and procainamide in the form of two distinct formulations to be separately administered.

4. Pharmaceutical combination according to the preceding claims, wherein the weight ratio between the platinum complex and Pd.HCl is comprised between 1:15 and 1:2, preferably between 1:10 and 1:4, most preferably about 1:6.

5. Pharmaceutical combination according to claim 3, wherein cisplatin is in the form suited to parenteral or intracavitary administration.

6. Pharmaceutical combination according to claim 3, wherein procainamide is in the form suited to parenteral or intracavitary or oral administration.

7. Pharmaceutical combination according to claim 5, wherein cisplatin is in the form of aqueous or saline solution.

8. Pharmaceutical combination according to claim 6, wherein procainamide is in the form of aqueous or saline solution, tablet, sugar-coated pill, gelatine capsule, syrup, drinkable ampoule, solution.

9. Pharmaceutical combination according to anyone of the preceding claims, wherein the platinum complex is selected from the group consisting of carboplatin, tetraplatin, iproplatin, TNO-6-, DACH.

## Patentansprüche

1. Pharmazeutische Zubereitung mit antineoplastischer Aktivität, die Cisplatin oder andere Antitumorplatinkomplexe und Procainamid enthält.

2. Pharmazeutische Zubereitung nach Anspruch 1, dadurch **gekennzeichnet,** daß sie in Form einer wäßrigen Lösung oder einer Salzlösung, die auf parenteralem oder intrakavitärem Weg verabreicht werden kann, vorliegt.

3. Pharmazeutische Zubereitung nach Anspruch 1, dadurch **gekennzeichnet,** daß sie in Form eines Kits vorliegt, das Cisplatin und Procainamid in Form von zwei unterschiedlichen Formulierungen, die getrennt verabreicht werden, enthält.

4. Pharmazeutische Zubereitung nach einem der vorhergehenden Ansprüche, dadurch **gekennzeichnet,** daß das Gewichtsverhältnis zwischen dem Platinkomplex und Pd.HCl zwischen 1:15 und 1:2, bevorzugt zwischen 1:10 und 1:4, am meisten bevorzugt bei etwa 1:6 liegt.

5. Pharmazeutische Zubereitung nach Anspruch 3, dadurch **gekennzeichnet,** daß das Cisplatin in Form einer für die parenterale oder intrakavitäre Verabreichung geeigneten Form vorliegt.

6. Pharmazeutische Zubereitung nach Anspruch 3, dadurch **gekennzeichnet,** daß das Procainamid in Form einer für die parenterale oder intrakavitäre oder orale Verabreichung geeigneten Form vorliegt.

7. Pharmazeutische Zubereitung nach Anspruch 5, dadurch **gekennzeichnet,** daß das Cisplatin in Form einer wäßrigen Lösung und einer Salzlösung vorliegt.

8. Pharmazeutische Zubereitung nach Anspruch 6, dadurch **gekennzeichnet,** daß das Procainamid in Form einer wäßrigen Lösung oder Salzlösung, als Tabletten mit zuckerbeschichteten Pillen, Gelantinekapseln, Sirup, trinkbare Ampullen, als Lösung vorliegt.

9. Pharmazeutische Zubereitung nach einem der vorgehenden Ansprüche, dadurch **gekennzeichnet,** daß der Platinkomplex ausgewählt wird aus der Gruppe, bestehend aus Carboplatin, Tetraplatin, Iproplatin, TNO-6-, DACH.

## Revendications

1. Composition pharmaceutique présentant une activité antinéoplastique, qui contient du cisplatine ou d'autres complexes du platine antitumoraux et du procaïnamide.

2. Composition pharmaceutique suivant la revendication 1, sous la forme de solution aqueuse ou saline à administrer par voie parentérale ou à l'intérieur de cavités.

3. Composition pharmaceutique suivant la revendication 1, sous la forme d'une trousse contenant du cisplatine et du procaïnamide sous la forme de deux formulations distinctes à administrer séparément.

4. Composition pharmaceutique suivant les revendications précédentes, dans laquelle le rapport en poids du complexe du platine au Pd.HCl est compris entre 1:15 et 1:2, de préférence entre 1:10 et 1:4 et est plus avantageusement d'environ 1:6.

5. Composition pharmaceutique suivant la revendication 3, dans laquelle le cisplatine est sous une forme adaptée à l'administration parentérale ou à l'intérieur de cavités.

6. Composition pharmaceutique suivant la revendication 3, dans laquelle le procaïnamide est sous une forme adaptée à l'administration parentérale ou à l'intérieur de cavités ou orale.

7. Composition pharmaceutique suivant la revendication 5, dans laquelle le cisplatine est sous la forme de solution aqueuse ou saline.

8. Composition pharmaceutique suivant la revendication 6, dans laquelle le procaïnamide est sous la forme de solution aqueuse ou saline, de comprimé, de pilule enrobée de sucre, de gélule de gélatine, de sirop, d'ampoule buvable, de solution.

9. Composition pharmaceutique suivant l'une quelconque des revendications précédentes, dans laquelle le complexe de platine est choisi dans le groupe consistant en carboplatine, tétraplatine, iproplatin, TNO-6- ou DACH.
